# EUROPEAN PATENT SPECIFICATION

(11) **EP 2 119 469 B1**
(45) Date of publication and mention of the grant of the patent: **14.05.2014**
(21) Application number: 08704452.5
(22) Date of filing: 05.02.2008
(51) Int. Cl.: A61M 37/00

(54) **MICRONEEDLE DEVICE FOR DIAGNOSIS OF ALLERGY**
MIKRONADELVORRICHTUNG ZUR DIAGNOSE VON ALLERGIEN
DISPOSITIF À MICRO-AIGUILLES DESTINÉ AU DIAGNOSTIC D'ALLERGIES

(30) Priority: 06.02.2007 JP 2007026650
(43) Date of publication of application: 18.11.2009
(73) Proprietor: Hisamitsu Pharmaceutical Co., Inc., Tosu-shi, Saga 841-0017 (JP)
(72) Inventor: TOKUMOTO, Seiji, Tsukuba-shi Ibaraki 305-0856 (JP); MATSUDO, Toshiyuki, Tsukuba-shi Ibaraki 305-0856 (JP); KUWAHARA, Tetsuji, Tsukuba-shi Ibaraki 305-0856 (JP)
(74) Representative: Andrae Westendorp
(86) International application number: PCT/JP2008/051825
(87) International publication number: WO 2008/096732

(56) References cited:
- WO-A2-03/051284
- JP-A- 2002 535 100
- JP-A- 2004 028 900
- JP-B2- 2 506 543
- US-A1- 2005 261 632

## Description

### Technical Field

The present invention relates to a microneedle device for diagnosis of an allergy, to perform simple, quick, and clear skin allergy diagnosis.

### Background Art

Immune system normally offers protection against disease and the like. However, immune reaction causes a malicious reaction for the body in some cases. This is called allergy. The cause that triggers the reaction is called an allergen (antigen).

A contact allergy test generally includes the so-called patch test. In such a contact allergy test, a sheet for a contact allergy test is used, in which an antigen is placed on the surface of the sheet and the sheet is capable of being applied to the skin of a subject. The sheet is applied to the subject's skin. After a certain period of contact, the sheet is detached from the skin. Based on the skin color change, the presence of allergy and the degree of allergy are generally macroscopically judged.

The term "patch test" used herein refers to a diagnostic method that can be easily performed without causing bleeding and pain regardless of blemishing the epidermis of the skin or not.

The skin is composed of the outermost horny layer, epidermis, dermis, and subcutaneous connective tissue. The horny layer that is generally composed of a dead cell layer and lipid bilayer acts as a strong barrier against many substances. In the epidermis layer, there are antigen presenting cells called Langerhans cells, which possess immune function. Langerhans cells capture protein antigens that enter the skin, degrade the antigens internally, and present the peptide fragments on the MHC molecules. The MHC-peptide complexes migrate to the lower cortex layer of the regional lymph nodes through afferent lymph venule, and come into contact with T cells via interdigitating cells. The antigens are transmitted from the skin to the T_{H} cells that are present in the lymph nodes by the migration of Langerhans cells. Langerhans cells have MHC class II molecules that are required for antigen presentation to the T_{H} cells.

Because of the strong barrier function presented by the horny layer of the skin, the period of contact must be long enough in a patch test. The period of contact is thus generally set to 48 hours. Moreover, in the conventional contact allergy test, the skin color change is macroscopically judged. Therefore, it requires proficiency to make proper judgments, and there have been a problem with accuracy in determining the degree of allergy.

Moreover, since antigens are usually high molecular weight substances such as proteins, which have poor absorbability, recently external administration forms have been examined by using an iontophoresis and electroporation apparatus as described in Patent Document 1 and Patent Document 2 in order to raise the permeability of the antigens. However, there has not been any case of directly using such apparatus in an allergy patch test or a contact allergy test. More specifically, there is a tendency to avoid using an iontophoresis and electroporation apparatus in a contact allergy test for reasons of convenience, simplicity, quickness and skin irritation.

Above all, recently, there have been various advances in microneedles and the coating techniques associated therewith. Patent Document 3 discloses an interface equipped with microneedles, in which the skin piercing component has a coating, as a device to inoculate vaccine through skin. However, the Document does not disclose any example of using the interface in a contact allergy test.

In order to solve the above described problems such as the contact for a long period of time in a patch test due to the strong barrier function of the horny layer of the skin, and unclear skin color change, it is possible to directly administer an antigen subcutaneously with an injection needle or needle. However, it is obvious that this method poses problems considering burden on a subject because various antibodies need to be examined due to the nature of allergy diagnosis.

In addition, a skin reaction test has been formulated in the diagnosis of allergies against drugs such as antibiotics, diabetes, infectious diseases (such as tuberculosis, hepatitis, and HIV). The skin reaction test has been confirmed to be effective in the prick test or intracutaneous administration test. The prick test refers to a method by holding an intradermal needle vertically to the skin, and piercing the skin so it does not cause bleeding, and is a method for diagnosing immediate-type drug allergies. The method is safer than the intracutaneous administration test. However, the technique is complicated and time consuming, and also require proficiency of health care professionals. It also poses the risk of bleeding depending on the degree of piercing the skin with the needle. Thus, particular caution is required for patients who may be infected with infectious diseases in the method. Moreover, in the intracutaneous reaction test, allergens are directly injected intracutaneously. Therefore, the method requires administration technique. In addition, another problem of the method is causing pain, bleeding, and overreaction. In fact, the intracutaneous reaction test for antibiotics has been abolished.

For example, Patent Document 4 discloses a test strip holding one or multiple projecting lancet needles capable of piercing the skin to a certain degree, in which allergic substances are applied or adhered to a part of the adhesive surface of a piece of adhesive tape. The length of the lancets is in the range of 0.762 mm to 1.524 mm, clearly suggesting that they pass through the epidermis, where many antigen presenting cells possessing the immune function are present, and administer the substances to the dermis. Many problems remained with the method as did the prick test in terms of burden to a subject due to causing pain and efficient diagnosis of allergies.
Patent Document 1: Japanese Patent No. 2506543
Patent Document 2: Japanese Patent Laid-Open No. 2002-535100
Patent Document 3: Japanese Patent Laid-Open No. 2004-528900
Patent Document 4: Japanese Patent Laid-Open No. 58-131919

Furthermore, WO 03/051284 A2 discloses a microabrader for delivering a substance into skin, said microabrader comprising an abrading surface, wherein said abrading surface comprises frustoconical or frustopyramidal microprotrusions comprising at least one scraping edge and projecting from said abrading surface, and an effective amount of said substance.

### Disclosure of the Invention

### Problems to be Solved by the Invention

As described above, the conventional patch test generally takes 48 hours. The skin prick test and intracutaneous administration test are not simple and safe diagnostic methods, and many of them generate unclear skin color change. Moreover, metal has often been used as the material for microneedles as Patent Document 3 discloses. However, the metal itself serves as an antigen in diagnosis of allergies in many cases, posing the problem of difficulty in giving an accurate diagnosis. In addition, metallic material that is not likely to serve as an allergen such as titanium can be used. However, such material is expensive, and thus is not practically usable for diagnosis. There has been a demand for more inexpensive microneedles. Furthermore, the length of the lancets disclosed in Patent Document 4 is in the range of 0.762 mm to 1.524 mm, which is longer than that of the microneedles. This has led to the problem of incapability of giving an efficient judgment and causing pain and bleeding.

An object of the present invention is to provide an inexpensive microneedle device for diagnosis of an allergy, enabling one to perform a clear skin test with simple operation for a short period of time in the diagnosis of allergies.

### Means for Solving the Problems

The present inventors examined a method for percutaneous administration of allergens using rats that have acquired immunity. As a result, the rats showed the strongest allergic reaction by using microneedles and by applying coating on the microneedles. Moreover, the inventors found that the use of a non-metallic synthetic or natural resin material as the microneedle material enables one to perform a quick and accurate diagnosis. Based on these findings, the inventors have achieved the present invention.

Specifically, the object can be achieved by a microneedle device as further disclosed in claim 1 for diagnosis of an allergy, comprising a substrate, microneedles formed on the substrate at a density of 100 to 10000 needles per 1 cm², being capable of piercing the skin 50 µm to 500 µm deep, and allergen-holding means placed on the microneedles, holding at least one allergen.

The microneedles used herein is prepared by the use of a non-metallic synthetic or natural resin material.

The non-metallic synthetic or natural resin material can be a biodegradable polymer such as polylactic acid, polyglycolide, polylactic acid-co-polyglycolide, capronolactone, polyurethane, or polyanhydride, or a polysaccharide such as hyaluronic acid, pullulan, dextran, dextrin or chondroitin sulfate, or a non-degradable polymer such as polycarbonate, polymethacrylic acid, ethylenevinylacetate, polytetrafluoroethylene or polyoxymethylene.

The allergen can be a peptide(s) or protein(s).

The allergen-holding means can be a coating on the microneedle surface with the allergen by using a coating carrier.

The coating of the allergen can only applied to the tip of the microneedle surface.

The coating carrier can include a water soluble polymer having a hydroxy group.

The coating carrier can include a water soluble polymer having a hydroxy group, the polymer being selected from the group consisting of polyethylene oxide, polyhydroxymethylcellulose, polyhydroxypropylcellulose, polyhydroxypropylmethylcellulose, polymethylcellulose, dextran, polyethylene glycol, and polyvinyl alcohol.

The coating carrier can include a polysaccharide. The polysaccharide can include a water soluble polymer selected from the group consisting of pullulan, carmellose sodium, chondroitin sulfate, hyaluronic acid, dextran, and Gum arabic.

The coating can be in the state of being anchored to the microneedle surface.

### Effect of the Invention

In contrast to the conventional allergy patch test that generally takes 48 hours, the microneedle device for diagnosis of the present invention enables one to perform a clearer skin test with simple operation in a short period of time such as approximately 20 minutes. There can be also provided an inexpensive microneedle device for diagnosis of an allergy, in which the device can cause less pain and practically avoid causing bleeding.

### Brief Description of the Drawings

Figure 1 is a diagram that shows an example of the microneedle device for diagnosis of an allergy of the present invention, in which (a) is a perspective view, and (b) is an A-B cross sectional view of (a); and
Figure 2 is a graph showing the evaluation results of Evans blue leakage.

### Best Mode for Carrying Out the Invention

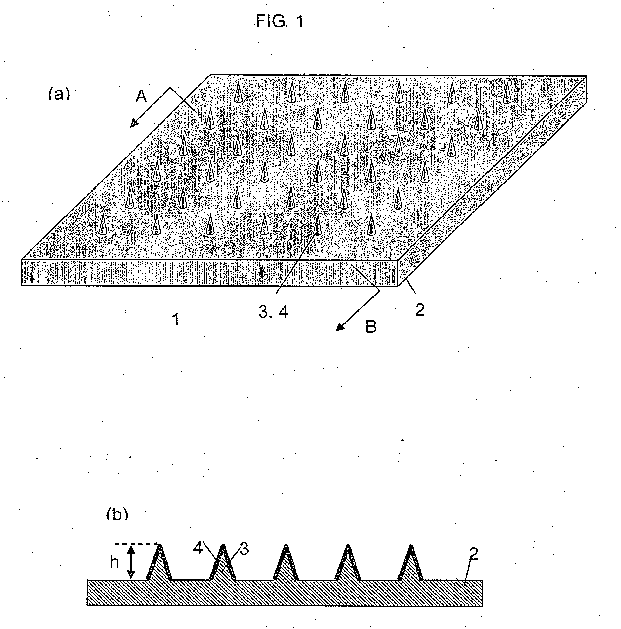

Figure 1 is a diagram that shows an example of the microneedle device for diagnosis of an allergy of the present invention, in which (a) is a perspective view, and (b) is an A-B cross sectional view of (a). As shown in Figure 1 (a), the microneedle device 1 of the present invention possesses a microneedle substrate 2 and a plurality of microneedles 3 that are 2-dimensionally placed on the microneedle substrate 2 and are capable of piercing the skin. On the microneedles 3, coating 4 is applied as an allergen-holding means that holds at least one allergen by using a coating carrier. The coating 4 is preferably in a state of being anchored to the surface of the microneedles 3. Alternatively, the coating 4 may be placed on the microneedle substrate 2 on which the microneedles are formed although it is not described in the drawing. The microneedles 3 are preferably prepared by the use of a non-metallic synthetic or natural resin material. Moreover, the shape of the microneedles 3 is in a circular cone shape in the present example. However, it is to be understood that the present invention is not limited thereto, and the shape can be a polygonal pyramid such as quadrangular pyramid or can be another shape.

The microneedle device comprises needle parts (microneedles) that are to be pierced into the skin or mucous membrane and a substrate that supports the needle part. A plurality of needle structures is aligned on the substrate. In the present invention, the needle structures of the microneedles are microstructure. Therefore, the length (height h) of the microneedles is preferably in the range of 50 µm to 500 µm. More specifically, the reason for setting the length of the microneedles to 50 µm or more is to ensure percutaneous administration of an allergen. The reason for setting the length of the microneedles to 500 µm or less is to prevent the microneedles from touching the nerves in order to steadily reduce the possibility of pain as well as unfailingly avoid the possibility of bleeding. Moreover, when the length thereof is 500 µm or less, the amount of the antigen entering the skin can be efficiently administered.

The microneedles used herein refer to convex structures and refer to needle-shaped objects or structures including needle-shaped objects in a broad sense. When the microneedles have a circular cone structure, the diameter of the base is generally in the range of 50 to 200 µm.

Furthermore, the shape is not limited to a simple needle-shaped object and may include blunt-pointed needles. Thus, the microneedles used herein are not limited to sharp-pointed needles.

The microneedle substrate is a substrate that supports the microneedles. The shape thereof includes, but is not limited to, a substrate equipped with a hole passing therethrough, making it possible to administer an antigen from the backside of the substrate. Examples of the material for the microneedles or substrate include silicon, silicon dioxide, ceramic, metal (such as stainless steel, titanium, nickel, molybdenum, chromium, and cobalt), and synthetic or natural resin material. Considering the antigenicity of microneedles and unit price of material, it is particularly preferred to use a biodegradable polymer such as polylactic acid, polyglycolide, polylactic acid-co-polyglycolide, pullulan, capronolactone, polyurethane or polyanhydride, or a non-degradable polymer polycarbonate, or a synthetic or natural resin material such as polymethacrylic acid, ethylenevinylacetate, polytetrafluoroethylene or polyoxymethylene. Moreover, it is preferred to use a polysaccharide such as hyaluronic acid, pullulan, dextran, dextrin or chondroitin sulfate.

The space between the rows is set to give the density of the microneedles typically at approximately 1 or 10 per 1 millimeter (mm) in a row of the needles. Generally, the rows are spaced at virtually equal intervals to the space of the needles aligned in the row, and have the density at 100 to 10000 needles per 1 cm². When the density is 100 needles or more, the amount of the antigen entering the skin can be efficiently administered. When the density is 10000 needles or more, it becomes difficult to give the strength of microneedles to be capable of piercing the skin.

Examples of method for manufacturing the microneedles include wet etching processing or dry etching processing using a silicon substrate, precision machining using metal or resin (such as discharge machining, laser machining, dicing processing, hot embossing, and injection molding), and mechanical cutting. With such processing method, the needle part and the support part are molded into one piece. Example of method of hollowing the needle part includes a method of performing secondary processing by using laser machining and the like after the needle part is being prepared.

The present invention enables the coating of the microneedles with an antigen by using purified water and/or a high molecular weight coating carrier, or purified water and/or a low molecular weight coating carrier. This feature ensures higher permeability for high molecular weight compounds with poor absorbability. More specifically, the entire surface of microneedles or a part thereof are coated with a coating agent selected from high molecular weight coating carriers such as polyethylene oxide, polyhydroxymethylcellulose, polyhydroxypropylcellulose, polyhydroxypropylmethylcellulose, polymethylcellulose, dextran, polyethylene glycol, and polyvinyl alcohol, low molecular weight coating carriers such as salts including sodium chloride and the like and sugar including glucose and the like. Subsequently, the microneedles are dried. When the microneedles are applied, the horny layer of the skin is pierced with the coated microneedles. By doing this, the antigen released from the coating passes through the pierced pores, and is absorbed percutaneously. In this case, the coating can be dissolved by the body fluid in the skin. Alternatively, a liquid for dissolving the coating may be applied separately to the site of application on the skin or to the coating itself.

Preferred coating carriers are high molecular weight coating carriers. Among them, more preferred are water soluble polymers having a hydroxy group such as polyethylene oxide, polyhydroxymethylcellulose, polyhydroxypropylcellulose, polyhydroxypropylmethylcellulose, polymethylcellulose, dextran, polyethylene glycol, polyvinyl alcohol, pullulan, carmellose sodium, chondroitin sulfate, hyaluronic acid, and Gum arabic. Particularly preferred is polyvinyl alcohol. The degree of saponification of polyvinyl alcohol is in the range of 78 to 100 mol%. Particularly preferred is a grade with a relatively low degree of saponification. The degree of saponification of a partially or intermediately saponified grade, such as PVA-220 (manufactured by KURARAY CO., LTD.), is approximately 94 mol%. Moreover, the average degree of polymerization of polyvinyl alcohol is in the range of 200 to 5000. Preferably, the average degree of polymerization in the range of 500 to 2000 is more effective because it has a relatively high solubility.

Furthermore, in case of considering the compatibility (property of being mixed homogeneously) with an allergen with a relatively high molecular weight, preferred are polysaccharide carriers such as polyhydroxymethylcellulose, hydroxypropylcellulose, polyhydroxypropylmethylcellulose, polymethylcellulose, dextran, polyethylene glycol, pullulan, carmellose sodium, chondroitin sulfate, hyaluronic acid, dextran, and Gum arabic. More preferred are hydroxypropylcellulose, pullulan, and Gum arabic. Particularly, most preferred are hydroxypropylcellulose (HPC-SSL (molecular weight: 15,000 to 30,000), HPC-SL (molecular weight: 30,000 to 50,000), HPC-L (molecular weight: 55,000 to 70,000), HPC-M (molecular weight: 110,000 to 150,000), HPC-H (molecular weight: 250,000 to 400,000)), pullulan, and hyaluronic acid.

The content of the coating carrier in the coating agent is in the range of 1 to 70% by weight, preferably in the range of 1 to 40% by weight, and particularly preferably in the range of 3 to 25% by weight. Moreover, the coating carrier may need to have viscosity to a degree that the coating agent will not cause a liquid drop. The required viscosity is approximately in the range of 100 to 100000 cps. More preferred viscosity is in the range of 500 to 50000 cps, and most preferred viscosity is in the range of 5000 to 30000 cps.

The thickness of coating on the microneedles is less than 50 µm, most preferably less than 25 µm and, more specifically, in the range of 1 to 10 µm. Generally, the thickness of coating is the average thickness measured across the surface of the microneedles after drying. Generally, the thickness of coating can be increased by applying a plurality of coatings of the coating carrier, and a coating can be formed by drying the coating during the intervals of consecutive coating. The coating agent is applied to the microneedles by using a known method, and the coating is anchored thereto by drying. Furthermore, the coating agent can be applied to the inner surface of the hollow channel of the needle structure of the microneedles and the bottom surface of the microneedle substrate. However, in terms of efficiency, it is enough to apply the coating to the needle part that actually enters the skin and, particularly, only to the tip part of the needles.

A liquid composition to be used for coating the microneedles is prepared by mixing a biocompatible carrier, a beneficial action substance to be transported and any coating auxiliary substance in some cases with a volatile liquid. The volatile liquid can be water, dimethylsulfoxide, dimethylformamide, ethanol, isopropylalcohol and a mixture thereof. The most preferred is water among them. The concentration of beneficial physiologically active substance in the liquid coating solution or suspension can typically be in the range of 0.1 to 65% by weight, preferably in the range of 1 to 30% by weight, and more preferably 3 to 20% by weight. The coating is particularly preferred to be in a state of being anchored. The term "the state of being anchored" refers to a state that the coating carrier is almost uniformly deposited on the subject. Immediately after coating, the coating carrier is anchored in the dry state by the known drying method such as air drying, vacuum drying, freeze drying and a combination thereof. However, after subcutaneous administration, the coating carrier is not always anchored in the dry state because it can hold moisture content or an organic solvent and the like that is in the equilibrium with the surrounding atmosphere.

The other known formulation auxiliary substances may be added to the coating as long as they do not have harmful effects on the required properties such as solubility and viscosity of the coating and on the physical integrity of the dried coating.

In a test for metal and chemical substance allergies, the corresponding metal and chemical substances can be used as allergens. In an allergy test to reveal the antigens for atopic dermatitis, house dust, acarine, fungi, bacteria and constituent compositions of foods and the like can be used as allergens. In case of pollen allergies, pollen of various species such as cedar, cypress, ragweed, mugwort, birch, rice plants can be used. Moreover, the antigen for the tuberculin reaction can also be used.

In case of food allergies, various food constituent components that are suspected to be involved in the onset and aggravation of the allergies such as eggs, milk, dairy products, meat, rice, beans, fish and fruits can be used.

Such constituent components can be used as crude products extracted by the general method or as a purified single component. In addition, products that are mass produced by introducing a gene to a microorganism or animal cell by applying generic engineering can also be used. Furthermore, degraded products, arbitrary fragment peptides of the antigens, or antigens in which a part of amino acids are mutated can also be used. Although these antigens can be used singularly, it is possible to use a method expecting a wider effect by a combination of two or more antigens.

### Examples

### (Example 1)

Male 12W hairless rats were intraperitoneally administered with protein lysozyme/complete Freund's adjuvant (1:1) (400 µg/rat). By allowing 12 days to pass, the rat model of anaphylaxis was established, in which the rats acquired immunity (IgE) against protein lysozyme.

Subsequently, under urethane anesthetization, the rats were administered with a 3% Evans blue (EB) solution (100 µL/100 g body weight) via the carotid artery. Protein lysozyme was then administered to the right and left abdomen by the methods described in Comparative Examples 2 through 4 and Examples 1 and 2 below. Protein lysozyme was not administered in Comparative Example 1.

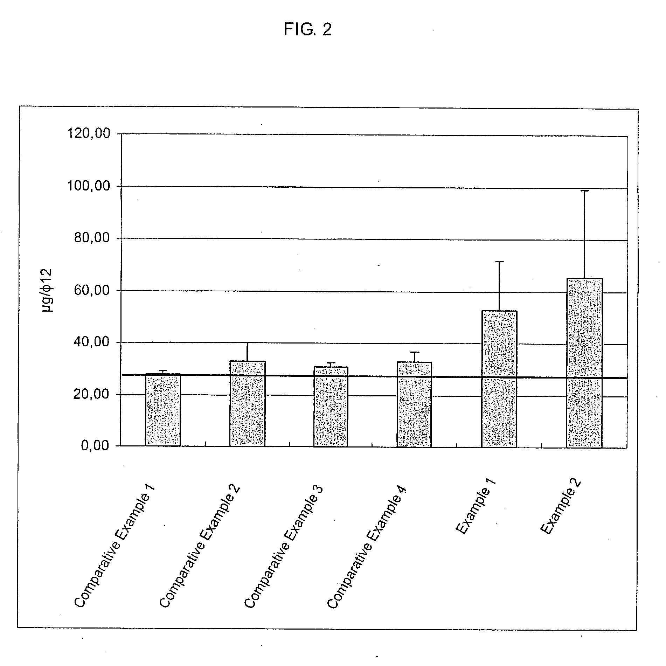

In addition, the evaluation of Evans blue coloring was conducted by the following procedure. More specifically, the site of administration on the skin was peeled and punched with a skin punch with an inner diameter of 12 mm (φ 12) to take out the skin section. The skin section was dissolved with 1 mL of 1 N potassium hydroxide and left to stand at 37°C overnight. To the dissolved skin section, 2.5 mL of a 0.6 N phosphoric acid solution and 6.5 mL of acetone were added. Subsequently, the mixture was centrifuged (1100* g, 20 minutes). Two hundred µL of the supernatant was collected and measured with a plate reader (spectrophotometer). Figure 2 shows the evaluation results of Evans blue leakage.

### (Comparative Example 1)

Protein lysozyme in the procedure of Example 1 was not administered. Instead, Comparative Example 1 was prepared by dripping 20 µL of a saline solution.

### (Comparative Example 2)

Protein lysozyme in the procedure of Example 1 was administered by the following method. The uncoated microneedles (made of polylactic acid, length 250 µm, density 841 needles/cm²) were used to pierce for 5 seconds. Subsequently, an unwoven fabric formulation (area 1 cm²) impregnated with a 5% protein lysozyme solution (400 µL) was patched for 10 minutes.

### (Comparative Example 3)

Protein lysozyme in the procedure of Example 1 was intracutaneously administered by the following method. Twenty µL of a 5% protein lysozyme solution was dripped onto the uncoated microneedles (made of polylactic acid, length 250 µm, density 841 needles/cm²). The microneedle device was compressed and patched for 10 minutes.

### (Comparative Example 4)

In the procedure of Example 1, 25 µL of a 0.5% protein lysozyme was intracutaneously administered to prepare Comparative Example 4.

### (Example 1)

To a 15% solution of polyvinyl alcohol (PVA220: manufactured by KURARAY CO., LTD.), protein lysozyme was dissolved to achieve the concentration of 5%. The solution was used to coat the microneedles (made of polylactic acid, length 250 µm, density 841 needles/cm²) three consecutive times by using a metal mask, and the microneedles were dried. Subsequently, the microneedle device was compressed and patched for 10 minutes.

### (Example 2)

Protein lysozyme of Example 1 was dissolved to achieve the concentration of 10% to prepare Example 2.

In the graph of Figure 2 that shows the evaluation results of Evans blue leakage, the value of Comparative Example 1 is shown as a horizontal line. As the graph of Figure 2 indicates, the degree of Evans blue coloring in Examples 1 and 2 were obviously higher than those of Comparative Examples. This suggests that the administration of protein lysozyme (the antigen) using the coating to the microneedles induces an allergy reaction more effectively.

### (Example 3) Test to confirm compatibility of various polymers with BSA and OVA

### Operation procedure

Mixed aqueous solutions of various polymers and BSA or OVA were prepared according to the conditions outlined in Table 1 and Table 2 below. Compatibility was evaluated by confirming the occurrence of aggregation and the presence of phase separation after centrifugal deaeration (the centrifugation conditions are described in the tables) (homogeneous liquid state: marked with ○, and heterogeneous liquid state: marked with ×). In Table 1 and Table 2, the o mark signifies the ones having compatibility, and the × mark signifies the ones having no compatibility. In addition, the % notion signifies % by weight in the description hereinafter. The measurement of coating content was performed by measuring BSA or OVA content (deposit) after extraction with 1 mL of purified water following the coating by the method described in the above Figure 2. Moreover, the term "not available" refers to the fact that no deposition of the polymer to the needles was confirmed.

**(Table 1)**

| Polymer | Polymer concentration (%) | OVA concentration (%) | Compatibility (centrifugation conditions) | Coating content (µg) |
|---|---|---|---|---|
| Pullulan | 20 | 20 | ○ (15000 rpm×2 min) | 50 |
| Pullulan | 15 | 16.7 | ○ (15000 rpm×2 min) | 16 |
| Pullulan | 7.5 | 16.7 | × | - |
| Pullulan | 5 | 16.7 | × | - |
| Hydroxypropylcellulose-SSL | 15 | 16.7 | × | - |
| Hydroxypropylcellulose-SSL | 20 | 16.7 | × | - |
| Hydroxypropylcellulose-SSL | 25 | 16.7 | × | - |
| Hydroxypropylcellulose-SL | 25 | 25 | ○ (15000 rpm×2 min) | 41 |
| Hydroxypropylcellulose-SL | 15 | 30 | × | - |
| Hydroxypropylcellulose-L | 13.3 | 16.7 | ○ (3000 rpm×2 min) | 9 |
| Hydroxypropylcellulose-L | 16.7 | 16.7 | ○ | 16 |
| Hydroxypropylcellulose-L | 20 | 16.7 | × | - |
| Hydroxypropylcellulose-L | 16 | 20 | × | - |
| Hydroxypropylcellulose-L | 13.3 | 20 | × | - |
| Hydroxypropylcellulose-L | 15 | 30 | × | - |
| Hydroxypropylcellulose-H | 4 | 16.7 | × | - |
| Hydroxypropylcellulose-H | 3 | 16.7 | ○ (5000 rpm×2 min) | 6 |
| Hydroxypropylcellulose-H | 2 | 16.7 | ○ (5000 rpm×2 min) | 5 |
| Hydroxypropylcellulose-H | 1.5 | 16.7 | ○ (5000 rpm×2 min) | - |
| Hydroxypropylcellulose-H | 1 | 16.7 | ○ (5000 rpm×2 min) | 1 |
| Methylcellulose (SM-25) | 7.5 | 16.7 | × | - |
| Methylcellulose (SM-25) | 4 | 16.7 | × | - |
| Methylcellulose (SM-25) | 2 | 16.7 | × | - |
| Methylcellulose (SM-400) | 5 | 16.7 | × | - |
| Methylcellulose (SM-400) | 3 | 16.7 | ○ (5000 rpm×2 min) | 7 |
| Methylcellulose (SM-400) | 1 | 16.7 | × | - |
| Methylcellulose (SM-8000) | 2.7 | 16.7 | × | - |
| Methylcellulose (SM-8000) | 4 | 16.7 | × | - |
| Methylcellulose (SM-8000) | 3 | 16.7 | × | - |
| Methylcellulose (SM-8000) | 2 | 16.7 | ○ (15000 rpmx2 min) | 3 |
| Methylcellulose (SM-8000) | 1 | 16.7 | × | - |
| Hyaluronic acid (MW900000) | 4 | 16.7 | ○ (15000 rpmx2 min) | 2 |
| Hyaluronic acid (MW900000) | 3 | 16.7 | ○ (15000 rpm×2 min) | 4 |
| Hyaluronic acid (MW900000) | 2 | 16.7 | ○ (15000 rpm×2 min) | 4 |
| Hyaluronic acid (MW900000) | 1 | 16.7 | ○ (15000 rpm×2 min) | 3 |
| Hyaluronic acid (MW2000000) | 2 | 16.7 | × | - |
| Partially neutralized polyacrylate (NP-600) | 3 | 16.7 | ○ (15000 rpmx2 min) | Not available |
| Partially neutralized polyacrylate (NP-600) | 1.5 | 16.7 | ○ (15000 rpm×2 min) | Not available |
| Partially neutralized polyacrylate (NP-800) | 3 | 16.7 | ○ (15000 rpmx2 min) | Not available |
| Partially neutralized polyacrylate (NP-800) | 1.5 | 16.7 | ○ (15000 rpm×2 min) | Not available |
| Polyethyleneglycol-20000 | 20 | 16.7 | × | - |
| Polyethyleneglycol-20000 | 10 | 16.7 | × | - |
| Polyvinylalcohol | 10 | 16.7 | × | - |
| Polyvinylalcohol | 5 | 16.7 | × | - |

**(Table 2)**

| Polymer | Polymer concentration (%) | BSA concentration (%) | Compatibility | Coating content (µg) |
|---|---|---|---|---|
| Pullulan | 15 | 30 | ○ (15000 rpmx2 min) | 30 |
| Pullulan | 20 | 20 | ○ (15000 rpmx2 min) | 18 |
| Hydroxypropylcellulose-SSL | 35 | 16.7 | × | - |
| Hydroxypropylcellulose-SSL | 20 | 16.7 | × | - |
| Hydroxypropylcellulose-SSL | 10 | 16.7 | × | - |
| Hydroxypropylcellulose-SSL | 37.5 | 10 | × | - |
| Hydroxypropylcellulose-SL | 25 | 20 | ○ (15000 rpm×2 min) | 20 |
| Hydroxypropylcellulose-SL | 20 | 16.7 | × | - |
| Hydroxypropylcellulose-SL | 16.7 | 16.7 | × | - |
| Hydroxypropylcellulose-L | 15 | 10 | × | - |
| Hydroxypropylceilulose-L | 20 | 16.7 | × | - |
| Hydroxypropylcellulose-L | 16.7 | 16.7 | × | - |
| Hydroxypropylcellulose-L | 13.3 | 16.7 | × | - |
| Hydroxypropylcellulose-M | 5 | 16.7 | × | - |
| Hydroxypropylcellulose-M | 3 | 16.7 | × | - |
| Hydroxypropylcellulose-M | 1 | 16.7 | × | - |
| Hydroxypropylcellulose-H | 3 | 16.7 | × | - |
| Hydroxypropylcellulose-H | 2 | 16.7 | × | - |
| Hydroxypropylcellulose-H | 1 | 16.7 | × | - |
| Methylcellulose (SM-25) | 4 | 16.7 | × | - |
| Methylcellulose (SM-25) | 2 | 16.7 | × | - |
| Methylcellulose (SM-25) | 1 | 16.7 | × | - |
| Methylcellulose (SM-400) | 5 | 167 | × | - |
| Methylcellulose (SM-400) | 3 | 16.7 | × | - |
| Methylcellulose (SM-400) | 1 | 16.7 | × | - |
| Methylcellulose (SM-8000) | 3 | 16.7 | × | - |
| Methylcellulose (SM-8000) | 2 | 16.7 | × | - |
| Methylcellulose (SM-8000) | 1 | 16.7 | × | - |
| Dextran (MW40000) | 50 | 11.4 | ○ (15000 rpm×2 min) | - |
| Dextran (MW70000) | 37.5 | 10 | ○ (15000 rpm×2 min) | - |
| Hyaluronic acid (MW900000) | 3 | 16.7 | ○ (15000 rpm×2 min) | - |
| Hyaluronic acid (MW900000) | 2 | 16.7 | ○ (15000 rpm×2 min) | - |
| Hyaluronic acid (MW900000) | 1 | 16.7 | ○ (15000 rpm×2 min) | - |
| Hyaluronic acid (MW900000) | 2.7 | 13.3 | ○ (15000 rpm×2 min) | - |
| Partially neutralized polyacrylate (NP-600) | 3 | 16.7 | ○ (15000 rpm×2 min) | Not available |
| Partially neutralized polyacrylate (NP-600) | 1.5 | 16.7 | ○ (15000 rpm×2 min) | Not available |
| Partially neutralized polyacrylate (NP-800) | 3 | 16.7 | ○ (15000 rpm×? min) | Not available |
| Partially neutralized polyacrylate (NP-800) | 1.5 | 16.7 | ○ (15000 rpm×? min) | Not available |
| Hydroxypropylmethylcellulose (90SH-30000) | 3.75 | 10 | × | - |
| Hydroxypropylmethylcellulose (90SH-30000) | 2.5 | 20 | × | - |
| Hydroxypropylmethylcellulose (65SH-1500) | 3.75 | 10 | × | - |
| Hydroxypropylmethylcellulose (65SH-1500) | 2.5 | 20 | × | - |
| Polyvinylpyrrolidone (K29/32) | 35 | 20 | × | - |
| Polyvinylpyrrolidone (K29/32) | 52.5 | 10 | × | - |
| Polyvinylpyrrolidone (K90) | 15 | 20 | × | - |
| Polyvinylpyrrolidone (K90) | 22.5 | 10 | × | - |
| Polyvinylpyrrolidone (K90) | 10 | 13.3 | × | - |
| Polyvinylpyrrolidone (K90) | 24 | 8 | × | - |
| Polyvinylpyrrolidone (K90) | 10 | 26.7 | × | - |
| Polyvinylpyrrolidone (K90) | 6 | 32 | × | - |
| Hydroxymethylcellulose (TC-5) | 15 | 5 | × | - |
| Hydroxymethylcellulose (TC-5) | 10 | 20 | × | - |
| Polyethyleneglycol-20000 | 20 | 16.7 | × | - |
| Polyethyleneglycol-20000 | 10 | 16.7 | × | - |
| Polyvinylalcohol | 10 | 16.7 | × | - |
| Polyvinylalcohol | 5 | 16.7 | × | - |
| Carmellose Na | 7.5 | 10 | × | - |
| Chondroitin sulfate | 30 | 10 | × | - |

Tables 1 and 2 show the results of compatibility of OVA or BSA and each water soluble polymer and the BSA or OVA content when used for coating the microneedles. Pullulan, hydroxypropylcellulose (HPC), methylcellulose, hyaluronic acid, and polyacrylate Na showed high compatibility by optimizing the composition ratio of the physiologically active substance and water soluble polymer. Particularly, pullulan showed high compatibility with OVA in high concentration. Moreover, when the solutions were used to perform the coating of microneedles by the method described in Figure 2, pullulan showed the highest value, followed in order by hydroxypropylcellulose (SL), methylcellulose, and hyaluronic acid. Hydroxypropylcellulose showed a difference in the amount of coating according to its grades. The values showed a tendency to descend in the order of HPC-SL > HPC-L > HPC-H. The reason for this is suspected to be that the viscoelasticity (viscosity) of hydroxypropylcellulose showed a tendency to rise as the molecular weight of the polymer was lowered, resulting in the elevation of deposition to the microneedles. In addition, methylcellulose showed favorable compatibility with OVA, but did not show favorable conditions with BSA. Although hyaluronic acid showed favorable compatibility with both OVA and BSA, it had poor viscosity and failed to give a sufficient coating amount. Although polyacrylate Na showed favorable compatibility, no deposition to the needles was confirmed. This proved it to be unsuitable as a coating carrier. Based on the results, by using a coating carrier that has compatibility with a high molecular weight physiologically active substance, there can be achieved a coating, in which the high molecular weight physiologically active substance is virtually uniformly included.

Furthermore, the following various polymers were used. Methylcellulose (SM-25, SM-400, and SM-8000) manufactured by Shin-Etsu Chemical Co., Ltd., polyacrylate (NP-600 and NP-800) manufactured by Showa Denko K.K., hydroxypropylmethylcellulose (90SH-30000, 65SH-1500, and TC-5) manufactured by Shin-Etsu Chemical Co., Ltd., and polyvinylpyrrolidone (K29/32 and K90) manufactured by Nippon Shokubai Co., Ltd., were used respectively.

### Industrial Applicability

According to the present invention described above, it is enabled to perform a clearer skin test with simple operation for a short period of time. There can also be provided inexpensive microneedles for diagnosis of an allergy. This would be a significant contribution to the development of medical industry and the related industries thereof.

## Claims

1. A microneedle device (1) for diagnosis of an allergy, comprising:
a substrate (2),
microneedles (3) formed on the substrate (2) at a density of 100 to 10000 needles per 1 cm², being capable of piercing the skin 50 µm to 500 µm deep, and
allergen-holding means placed on the microneedles (3), holding at least one allergen,
wherein the microneedles (3) are prepared by the use of a non-metallic synthetic or natural resin material.

2. The microneedle device (1) for diagnosis of an allergy according to Claim 1, wherein the non-metallic synthetic or natural resin material is a biodegradable polymer such as polylactic acid, polyglycolide, polylactic acid-co-polyglycolide, capronolactone, polyurethane, or polyanhydride; or hyaluronic acid, pullulan, dextran, dextrin or chondroitin sulfate that are all polysaccharides; or polycarbonate, polymethacrylic acid, ethylenevinylacetate, polytetrafluoroethylene or polyoxymethylene that are all non-degradable polymers.

3. The microneedle device (1) for diagnosis of an allergy according to any of claims 1 to 2, wherein the allergen is a peptide(s) or a protein(s).

4. The microneedle device (1) for diagnosis of an allergy according to any of claims 1 to 3, wherein the allergen-holding means is a coating (4) on the microneedle surface with the allergen by using a coating carrier.

5. The microneedle device (1) for diagnosis of an allergy according to claim 4, wherein the coating carrier includes a water-soluble polymer having a hydroxy group.

6. The microneedle device (1) for diagnosis of an allergy according to claim 5, wherein the coating carrier includes a water-soluble polymer having a hydroxy group, the polymer being selected from the group consisting of polyethylene oxide, polyhydroxymethylcellulose, polyhydroxypropylcellulose, polyhydroxypropylmethylcellulose, polymethylcellulose, dextran, polyethylene glycol, and polyvinyl alcohol.

7. The microneedle device (1) for diagnosis of an allergy according to claim 5, wherein the coating carrier includes a polysaccharide.

8. The microneedle device (1) for diagnosis of an allergy according to claim 7, wherein the polysaccharide includes a water-soluble polymer selected from the group consisting of pullulan, carmellose sodium, chondroitin sulfate, hyaluronic acid, dextran, and Gum arabic.

9. The microneedle device (1) for diagnosis of an allergy according to any of claims 4 to 8, wherein the coating (4) is in a state of being anchored to the microneedle surface.

## Patentansprüche

1. Mikronadelvorrichtung (1) zur Diagnose einer Allergie, umfassend:
ein Substrat (2),
Mikronadeln (3), welche auf dem Substrat (2) in einer Dichte von 100 bis 10000 Nadeln pro cm² gebildet sind, die zum Eindringen in die Haut bis zu einer Tiefe von 50 µm bis 500 µm in der Lage sind, und
Allergen-Haltemittel, welche auf den Mikronadeln (3) platziert sind, die mindestens ein Allergen halten,
wobei die Mikronadeln (3) unter Verwendung eines nicht-metallischen synthetischen oder natürlichen Harzmaterials hergestellt sind.

2. Mikronadelvorrichtung (1) zur Diagnose einer Allergie gemäß Anspruch 1, wobei das nicht-metallische synthetische oder natürliche Harzmaterial ein bioabbaubares Polymer wie Polymilchsäure, Polyglycolid, Polymilchsäure-co-Polyglycolid, Capronolacton, Polyurethan oder Polyanhydrid; oder Hyaluronsäure, Pullulan, Dextran, Dextrin oder Chondroitinsulfat, welche alle Polysaccharide sind; oder Polycarbonat, Polymethacrylsäure, Ethylenvinylacetat, Polytetrafluorethylen oder Polyoxymethylen, welche alle nicht-abbaubare Polymere sind, ist.

3. Mikronadelvorrichtung (1) zur Diagnose einer Allergie gemäß einem der Ansprüche 1 bis 2, wobei das Allergen ein Peptid(e) oder ein Protein(e) ist.

4. Mikronadelvorrichtung (1) zur Diagnose einer Allergie gemäß einem der Ansprüche 1 bis 3, wobei das Allergen-Haltemittel ein Überzug (4) auf der Mikronadelvorrichtung mit dem Allergen unter Verwendung eines Überzugsträgers ist.

5. Mikronadelvorrichtung (1) zur Diagnose einer Allergie gemäß Anspruch 4, wobei der Überzugsträger ein wasserlösliches Polymer mit einer Hydroxygruppe einschließt.

6. Mikronadelvorrichtung (1) zur Diagnose einer Allergie gemäß Anspruch 5, wobei der Überzugsträger ein wasserlösliches Polymer mit einer Hydroxygruppe einschließt, wobei das Polymer aus der Gruppe ausgewählt ist, welche aus Polyethylenoxid, Polyhydroxymethylcellulose, Polyhydroxypropylcellulose, Polyhydroxypropylmethylcellulose, Polymethylcellulose, Dextran, Polyethylenglycol und Polyvinylalkohol besteht.

7. Mikronadelvorrichtung (1) zur Diagnose einer Allergie gemäß Anspruch 5, wobei der Überzugsträger ein Polysaccharid einschließt.

8. Mikronadelvorrichtung (1) zur Diagnose einer Allergie gemäß Anspruch 7, wobei das Polysaccharid ein wasserlösliches Polymer einschließt, welches aus der Gruppe ausgewählt ist, die aus Pullulan, Carmellose-Natrium, Chondroitinsulfat, Hyaluronsäure, Dextran und Gummiarabikum besteht.

9. Mikronadelvorrichtung (1) zur Diagnose einer Allergie gemäß einem der Ansprüche 4 bis 8, wobei der Überzug (4) verankert auf der Mikronadeloberfläche vorhanden ist.

## Revendications

1. Dispositif à micro-aiguilles (1) pour le diagnostic d'une allergie, comprenant :
un substrat (2),
des micro-aiguilles (3) formées sur le substrat (2) à une densité de 100 à 10 000 aiguilles par cm², capables de percer la peau sur une profondeur de 50 µm à 500 µm, et
un moyen de fixation d'allergène, placés sur les micro-aiguilles (3), fixant au moins un allergène,
où les micro-aiguilles (3) sont préparées par utilisation d'un matériau non métallique en résine synthétique ou naturelle.

2. Dispositif à micro-aiguilles (1) pour le diagnostic d'une allergie selon la revendication 1, où le matériau non métallique en résine synthétique ou naturelle est un polymère biodégradable tel que le poly(acide lactique), un polyglycolide, un poly(acide lactique)-co-polyglycolide, la capronolactone, un polyuréthanne, ou un polyanhydride ; ou l'acide hyaluronique, un pullulane, un dextrane, une dextrine ou le sulfate de chondroïtine, qui sont tous des polysaccharides ; ou un polycarbonate, le poly(acide méthacrylique), un copolymère éthylène-acétate de vinyle, le polytétrafluoroéthylène ou le polyoxyméthylène, qui sont tous des polymères non dégradables.

3. Dispositif à micro-aiguilles (1) pour le diagnostic d'une allergie selon la revendication 1 ou 2, où l'allergène est un ou des peptides ou protéines.

4. Dispositif à micro-aiguilles (1) pour le diagnostic d'une allergie selon l'une des revendications 1 à 3, où le moyen de fixation d'allergène est un revêtement (4) à la surface de la micro-aiguille avec l'allergène en utilisant un support de revêtement.

5. Dispositif à micro-aiguilles (1) pour le diagnostic d'une allergie selon la revendication 4, où le support de revêtement comprend un polymère soluble dans l'eau portant des radicaux hydroxy.

6. Dispositif à micro-aiguilles (1) pour le diagnostic d'une allergie selon la revendication 5, où le support de revêtement comprend un polymère soluble dans l'eau portant des radicaux hydroxy, le polymère étant choisi parmi le groupe consistant en le poly(oxyde d'éthylène), la polyhydroxyméthylcellulose, la polyhydroxypropylcellulose, la polyhydroxypropyl-méthylcellulose, la polyméthylcellulose, le dextrane, le polyéthylèneglycol et le poly(alcool vinylique).

7. Dispositif à micro-aiguilles (1) pour le diagnostic d'une allergie selon la revendication 5, où le support de revêtement comprend un polysaccharide.

8. Dispositif à micro-aiguilles (1) pour le diagnostic d'une allergie selon la revendication 7, où le polysaccharide comprend un polymère soluble dans l'eau, choisi parmi le groupe consistant en le pullulane, la carmellose sodique, le sulfate de chondroïtine, l'acide hyaluronique, le dextrane et la gomme arabique.

9. Dispositif à micro-aiguilles (1) pour le diagnostic d'une allergie selon l'une quelconque des revendications 4 à 8, où le revêtement (4) est dans un état tel qu'il est ancré à la surface de la micro-aiguille.
